# EUROPEAN PATENT APPLICATION

(11) **EP 4 623 878 A1**
(43) Date of publication of application: **01.10.2025**
(21) Application number: 24185097.3
(22) Date of filing: 27.06.2024
(51) Int. Cl.: A61F 13/15, A61F 13/494, A61F 13/495, A61F 13/496, A61F 13/49

(54) **METHOD AND APPARATUS FOR THE MANUFACTURE OF ABSORBENT ARTICLE WITH TRANSVERSAL BARRIER**

(30) Priority: 29.03.2024 EP 24167928
(71) Applicant: Ontex BV, 9255 Buggenhout (BE); Ontex Group NV, 9320 Erembodegem (BE)
(72) Inventor: IFFLAND, Dirk, D-45529 Hattingen (DE); BUYSSE, Michiel, 9968 Oosteeklo (BE); PSCHYKLENK, Lukas, 56761 Müllenbach (DE); FRANSEN, Markus, 56269 Dierdorf (DE)
(74) Representative: Saurat, Thibault

(57) **Abstract**

The present disclosure pertains to a method for the manufacture of an absorbent article (1) comprising the steps of:
• providing a front panel (2) comprising front and back transversal edges;
• providing a back panel (3) comprising front and back transversal edges;
• providing an absorbent insert (4) comprising front and back transversal edges and left and right longitudinal edges connecting the front and back transversal edges to form a perimeter thereof;
• folding a portion of the front panel (2) proximal to the front transversal edge and/or folding a portion of the back panel (3) proximal to the back transversal edge, about at least two folding lines (62,63) to form a folded arrangement (64),
• joining said absorbent insert (4) to said folded arrangement (64),
• unfolding a portion of the folded arrangement (64) and folding a portion of said absorbent insert (4) proximal to the front and/or back transversal edges thereof onto itself, preferably in the shape of a substantially U-fold, to form transversal waist barrier(s) (5); and
• joining said front and back panels (2, 3) together along a pair of oppositely disposed side seams.

The present disclosure also relates to an apparatus to manufacture an absorbent article (1) according to the present method and an absorbent article (1).

## Description

### TECHNICAL FIELD

The present disclosure is directed to an absorbent article for personal hygiene, typically in the form of pants (such as for babies, youths or adults) and generally of the disposable kind as well as the method for the manufacture of such absorbent articles and the apparatus to carry out such method.

### BACKGROUND

Absorbent articles for personal hygiene are designed to absorb and contain bodily exudates, such as a large quantity of urine and/or stool. Non-limiting examples of disposable absorbent articles include diapers, pants, training pants, pad, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp (also referred to as fluff pulp and/or cellulose fibers) with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP3451989 B1) directed to limiting the risk of leakage by providing transversal barriers at the front and/or back of a diaper. However whilst such executions may be effective on diaper constructs they are less effective and or complex/costly to incorporate into a pant concept.

An attempt to overcome such shortcomings has been made in for example WO2021/170027 A1 wherein a nonwoven layer of the front and/or back belt is folded a plurality of times to form a waist guard. Although this provides for an effective way to introduce a transversal barrier in a pant construct, there are several drawbacks that have been identified: firstly, the nonwoven that is generally hydrophobic may be sufficient to capture solids but is less effective to capture larger amounts of liquid exudates (particularly under pressure such as when a baby is moving and/or application of sudden pressure such as sitting/dropping on the floor on its buttocks); secondly it limits the positioning thereof to a closer position at the waist edge which is less effective in providing a barrier since it is positioned further away from the core and to position it closer to the core and away from the waist edge would require a significant increase in length of the nonwoven which adds significant waste and cost; thirdly applying the multiple folds at different positions of the same component and especially further elasticizing the region when/if desired may add significant process complexity particularly at high speeds.

A need therefore exists for improved absorbent articles having leakage protection particularly for substantially liquid exudates such as urine and/or liquid stool yet in in a cost-effective manner and with more limited additional waste as well as having an efficient method and apparatus for the manufacture of such absorbent articles.

Documents EP4321138A1 and EP4321140A1 describe such absorbent articles with the latter describing a method to manufacture such absorbent articles. While such method is perfectly adequate to manufacture such absorbent articles, it can still be further improved with respect to the stability of the absorbent articles during the manufacturing process, especially at higher production speeds.

### SUMMARY

The disclosure relates to an absorbent article for personal hygiene, preferably a disposable pant, comprising: a front panel preferably substantially transversely extending; a back panel preferably substantially transversely extending; and an absorbent insert, preferably substantially longitudinally extending, joined to each of said front and back panels, said absorbent insert comprising front and back transversal edges and left and right longitudinal edges connecting the front and back transversal edges to form a perimeter thereof and generally wherein the left and right longitudinal edges extend substantially along the longitudinal axis y and are oppositely disposed such that said longitudinal axis y extends therebetween, and the front and back transversal edges extend substantially along the transversal axis x, and typically substantially perpendicular to the longitudinal axis y, and are oppositely disposed such that said transverse axis x extends therebetween). The front and back panels are joined together to define a pair of side seams, and at least a portion of said absorbent insert proximal to the front and/or back transversal edges thereof comprises a transversal waist barrier comprising a substantially liquid impermeable film layer.

The disclosure pertains to a method for the manufacture of an absorbent article (1) comprising the steps of:
- providing a front panel comprising front and back transversal edges;
- providing a back panel comprising front and back transversal edges;
- providing an absorbent insert comprising front and back transversal edges and left and right longitudinal edges connecting the front and back transversal edges to form a perimeter thereof;
- folding a portion of the front panel proximal to the front transversal edge and/or folding a portion of the back panel proximal to the back transversal edge, about at least two folding lines to form a folded arrangement,
- joining said absorbent insert to said folded arrangement,
- unfolding a portion of the folded arrangement and folding a portion of said absorbent insert proximal to the front and/or back transversal edges thereof onto itself, preferably in the shape of a substantially U-fold, to form transversal waist barrier(s); and
- joining said front and back panels together along a pair of oppositely disposed side seams.

The front and/or back panels can be provide separately or as webs, meaning a continuous web(s) of front and/or back panels, the webs can then be cut into discrete articles downstream of the process.

This method is particularly well adapted for making such absorbent articles with transversal barriers, especially at high speeds, since the folding of the portion(s) of the absorbent inserts onto itself is carried out on a continuous web, namely the web of front and/or back panels, which stabilizes the folding process as it is done continuously.

Said folding step can occur directly after, meaning sequentially after, or subsequentially, the joining step, or the present disclosure encompasses embodiments with additional steps occurring between said joining step and said folding step, for example a step where adhesive is intermittently applied onto a portion of the absorbent insert, a step where said absorbent insert is conveyed downstream from said joining step to said folding step. The present disclosure also encompasses embodiments where the joining step and the folding step are partially concurrent, for example, the absorbent insert is started to be folded even though the absorbent insert is not entirely joined to the front and/or back panels. Preferably, said folding and unfolding steps are carried out by static folding or unfolding where a static element, *i.e.* a stator, such as a stationary rail or a stationary rod or a stationary folding blade, said static element deflects a portion of said folded arrangement or of said absorbent insert proximal to the front and/or back transversal edges thereof onto itself. In other words, the unfolding and folding steps are preferably done by passive folding meaning that the folding is done with a moving element, i.e. the web of front and/or back panels and absorbent insert, being folded by an unmoving, or immobile or fixed element, e.g. a rail or a rod or a plate or a blade. Such folding is more cost efficient and more reproducible. The present disclosure encompasses embodiments where the folding step is carried out by dynamic folding, where a mobile element, e.g. an actuated folding blade or folding rail, moves or is actuated to fold a portion of said front and/or back panels and of said portion of said absorbent insert proximal to the front and/or back transversal edges thereof onto itself. In other words, the method preferably comprises a step such as statically folding a portion of said absorbent insert proximal to the front and/or back transversal edges thereof onto itself, preferably in the shape of a substantially U-fold, to form transversal waist barrier(s).

According to an embodiment, said folding a portion of said absorbent insert proximal to the front and/or back transversal edges thereof onto itself, preferably in the shape of a substantially U-fold, to form transversal waist barrier(s) is substantially simultaneous or concurrent to unfolding a portion of the folded arrangement. Preferably, the unfolding of a portion of the folded arrangement leads to the folding of a portion of said absorbent insert proximal to the front and/or back transversal edges thereof onto itself. In other words, it is said unfolding, or opening of one of the fold, that results or bring about said folding of a portion of said absorbent insert.

By "unfolding" it is meant the opening of a fold. The folded arrangement comprises at least two folding lines and hence at least two folds being formed during said folding step forming the folded arrangement, by "unfolding a portion of the folded arrangement", it is meant the opening one of said fold formed during the folding step forming the folded arrangement.

According to an embodiment, the folded arrangement comprises a Z-shaped fold.

According to an embodiment, said Z-shaped fold comprises the transversal edge of the front and/or back panel and a first and second folding lines, the length between the first and second folding lines being greater than the length between the second folding line and the transversal edge of the front and/or back panel, preferably with respect to the cross direction.

By "length", it is meant a dimension or distance or spatial remoteness in relation to one direction, for example the cross direction, hence the length between the first and second folding lines being and the length between the second folding line and the transversal edge of the front and/or back panel are compared on one same direction.

According to an embodiment, the Z-shaped fold comprises a total length corresponding to the addition, or combination, of the length between the first and second folding lines and the length between the second folding line and the transversal edge of the front and/or back panel, preferably with respect to the cross direction, wherein the ratio between the total length and the length between the first and second folding lines is comprised between about 0,4 and 7,5, preferably between about 0,7 and 3,3, more preferably between about 1,2 and 2,1.

According to an embodiment, the method further comprises a step of folding a portion of the front panel proximal to the front transversal edge and/or folding a portion of the back panel proximal to the back transversal edge, about four folding lines to form a folded arrangement, the folded arrangement being in the form of an omega-shaped folded formation.

According to an embodiment, the method further comprises the step of arranging an elastic material onto or over the folded arrangement prior to step of joining said absorbent insert to said folded arrangement.

According to an embodiment, the method further comprises the step of intermittently applying adhesive onto said portion of the absorbent insert proximal to the front and/or back transversal edges.

According to an embodiment, prior to joining the absorbent insert to the folded arrangement, the method further comprises the step of applying adhesive onto the garment facing side of the absorbent insert, said adhesive being applied in two discrete regions.

According to an embodiment, the method further comprises the step of applying adhesive onto said portion of the absorbent insert proximal to the front and/or back transversal edges in an adhesive pattern, said adhesive pattern comprising at least two areas of adhesive arranged at the transversal extremities of the front and/or back transversal edges, a further area of adhesive extending transversally between said two areas of adhesive, said further area being at a longitudinal distance from the transversal edge, and an adhesive-free area arranged between the transversal edge of the absorbent insert and said further area.

According to an embodiment, the method further comprises the step of compressing the folded portion of the folded absorbent insert preferably at least the transversal waist barrier(s), once formed.

According to an embodiment, the method further comprises the step of strand coating the elastic material prior to the step of arranging said elastic material onto or over the folded arrangement.

According to an embodiment, the elastic material is arranged such that said elastic material is sandwiched between the absorbent insert and the outer nonwoven layer. More preferably, the absorbent insert comprises a backsheet and eventually an outer cover on the garment facing surface of the absorbent core, the elastic material being sandwiched between the backsheet and the outer nonwoven layer or the elastic material being sandwiched between the outer cover and the outer nonwoven layer or the elastic material being sandwiched between the backsheet and outer cover and the outer nonwoven layer e.g. in cases where the outer cover is transversally shorter than the backsheet. In other words, the elastic material is directly or indirectly joined to the backsheet, preferably by adhesive, and sandwiched between the backsheet and/or outer cover and the outer nonwoven layer. Preferably, this said step occurs prior to said folding step, meaning before a portion of said absorbent insert proximal to the front and/or back transversal edges thereof is folded onto itself.

According to an embodiment, the method further comprises the step of mechanically bonding the folded transversal edge of the absorbent insert once the transversal waist barrier(s) has been formed.

According to an embodiment, the method further comprising the step of compressing the folded portion of the folded absorbent insert, preferably at least the transversal waist barrier(s), once formed. According to an embodiment, the method further comprising the step of compressing the folded portion of the folded absorbent insert once the outer nonwoven layer has been joined directly or indirectly to the folded absorbent insert. According to an embodiment, the method further comprising the step of mechanically bonding the folded transversal edge of the absorbent insert once the transversal waist barrier(s) has been formed.

These embodiments can be taken alone or in combination.

The present disclosure also pertains to an apparatus for the manufacture of an absorbent article according to a method as described hereabove, wherein the apparatus comprises:
- a conveying device, preferably a conveyor belt or a plurality of rollers or drums, for conveying said front and/or back panels;
- a folding unit for folding a portion of the front panel proximal to the front transversal edge and/or folding a portion of the back panel proximal to the back transversal edge, about at least two folding lines to form a folded arrangement,
- an unfolding unit for unfolding a portion of the folded arrangement and folding a portion of said absorbent insert proximal to the front and/or back transversal edges thereof onto itself preferably in the shape of a substantially U-fold, to form transversal waist barrier(s), said absorbent insert being joined to said folded arrangement and to front and/or back panel;
- a bonding unit for joining the joining said front and back panels together along a pair of oppositely disposed side seams.

The present disclosure also pertains to an absorbent article, preferably as manufactured according to method as described hereabove, wherein said article comprises
- a substantially transversely extending front panel;
- a substantially transversely extending back panel; and
- a substantially longitudinally extending absorbent insert joined to each of said front and back panels, said absorbent insert comprising front and back transversal edges and left and right longitudinal edges connecting the front and back transversal edges to form a perimeter thereof;
- wherein the front and back panels are joined together to define a pair of side seams, and
- at least a portion of said absorbent insert proximal to the front and/or back transversal edges thereof comprises a transversal waist barrier comprising a substantially liquid impermeable film layer,
wherein the length between the transversal edge of the absorbent insert and transversal edge of the front and/or back panel once the transversal barrier has been formed is comprised between about 5 and 30 mm, preferably between about 6 and 25 mm, more preferably between about 8 and 20 mm, even more preferably between about 9 and 15 mm, preferably with respect to the cross direction.

Similarly, by "length", it is meant a dimension or distance or spatial remoteness in relation to one direction, for example the cross direction.

According to an embodiment, a portion of the front panel proximal to the front transversal edge and/or a portion of the back panel proximal to the back transversal edge comprises at least one folding lines, the shortest length between the transversal barrier and said folding line being comprised between about 20 and 100 mm, preferably between about 30 and 70 mm, more preferably between about 40 and 55 mm, preferably with respect to the cross direction.

### BRIEF DESCRIPTION OF THE FIGURES

**FIG. 1** is a top planar view of an absorbent article according to an embodiment of the present disclosure.
**FIG. 2** is a top planar view of an absorbent article according to an embodiment of the present disclosure.
**FIG. 3** is a partial cross-section of an absorbent article according to an embodiment of the present disclosure.
**FIG. 4** is a top planar view of an absorbent article according to an embodiment of the present disclosure.
**FIG. 5** is a top planar view of an absorbent article according to an embodiment of the present disclosure.
**FIG. 6A** is a top planar view of an absorbent article according to an embodiment of the present disclosure prior to the folding of the portion of said absorbent insert proximal to the back transversal edge thereof.
**FIG. 6B** is a top planar view of an absorbent article according to FIG. 6A after folding of the portion of said absorbent insert proximal to the back transversal edge thereof.
**FIG. 7** is a top planar view of an absorbent article according to an embodiment of the present disclosure.
**FIG. 8** is a top planar view of an absorbent article according to an embodiment of the present disclosure.
**FIG. 9** comprising FIG. 9A to FIG. 9F is a partial cross-section of an absorbent article according to the present disclosure and illustrates different steps of the method to manufacture absorbent articles with a transversal barrier.
**FIG. 10** comprising FIG. 10A and FIG. 10B is a partial cross-section of an absorbent article according to the present disclosure and illustrates different representations of the folded arrangement.
**FIG. 11** comprising FIG. 11A and FIG. 11B is a partial top planar view of an absorbent article seen from above according to the present disclosure and illustrates different steps of the method to make the folded arrangement.
**FIG. 12** comprising FIG. 12A to FIG. 12C is a partial cross-section of an absorbent article according to the present disclosure and illustrates different steps of the method to manufacture absorbent articles with a transversal barrier.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure. As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

"Front" and "rear or back" of the absorbent article as used herein typically refer for the "front" end of the article is that which is most proximal to the front of the subject when worn, the "rear or back" end of the article is that which is most proximal to the rear or back of the subject when worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or human-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

As used herein, the terms "machine direction", "cross direction" and "vertical direction" are with respect to the apparatus suitable for forming absorbent articles with transversal barrier(s) in an assembled state or in a final installation state, meaning once normally functioning. The term "in the direction" as used herein means along that direction, e.g. in the machine direction means along the machine direction.

The values indicated herein are measured according to the methods indicated herein below, unless specified otherwise. All measurements are performed at 21±2° C. and 50±20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

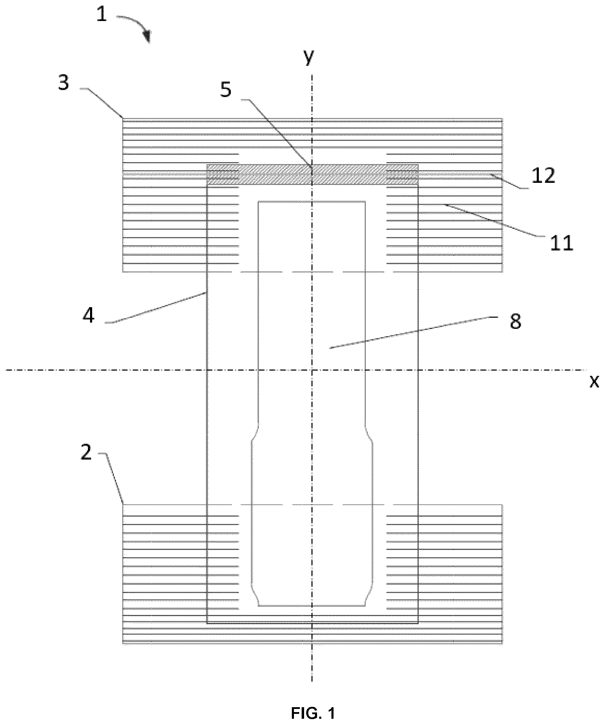

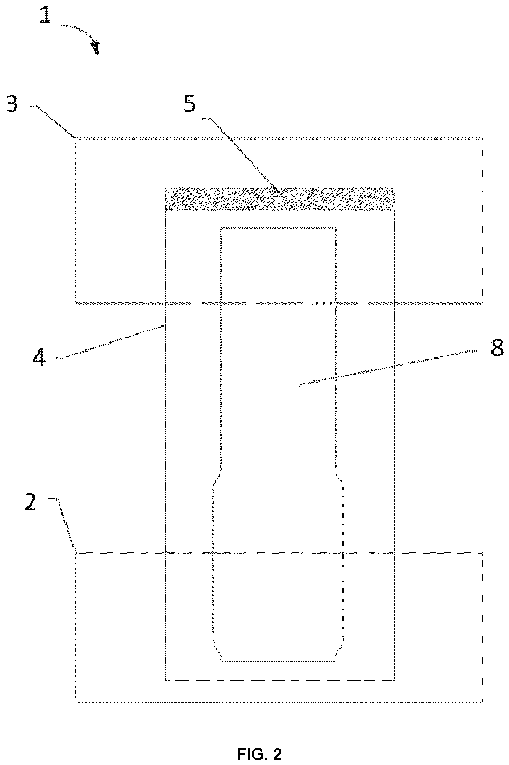

As exemplified in FIG. 1 to FIG. 3, absorbent articles herein are for personal hygiene, preferably in the form of a pant, the absorbent article comprising: a substantially transversely extending front panel (2); a substantially transversely extending back panel (3); and a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof (generally wherein the left and right longitudinal edges extend substantially parallel to the longitudinal axis y and are oppositely disposed such that said longitudinal axis y extends therebetween, and the front and back transversal edges extend substantially parallel to the transversal axis x, and typically substantially perpendicular to the longitudinal axis y, and are oppositely disposed such that said transverse axis x extends therebetween); wherein the front and back panels (2, 3) are joined together to define a pair of side seams, and wherein at least a portion of said absorbent insert (4) proximal to the front and/or back transversal edges thereof comprises a transversal waist barrier(s) (5) comprising a substantially liquid impermeable film layer, preferably wherein said transversal waist barrier (5) is an up-standing barrier generally arranged to block substantially liquid exudates from flowing therethrough. Advantageously, this arrangement allows for an absorbent article with superior leakage prevention particularly for substantially liquid exudates such as urine and/or liquid stool. Generally, the substantially liquid impermeable film layer is an integral component of at least one element or portion of the absorbent article such as a backsheet (7) thereof typically such that no additional and/or separate layer, element and/or material is incorporated to form the transversal waist barrier(s) (5). Advantageously this allows to reduce waste and/or cost associated with incorporating separate elements to create the transversal waist barrier(s).

The backsheet (7) is generally that portion of the absorbent article positioned adjacent the garment-facing surface of the absorbent core (8) and which prevents the exudates absorbed and contained therein from soiling articles such as bedsheets and undergarments. The backsheet (7) is typically impermeable to liquids (e.g. urine). The backsheet (7) may for example be or comprise a thin plastic film such as a thermoplastic film having a thickness of about 0.012 mm to about 0.051 mm. Preferably the basis weight of the backsheet (7) is less than or equal to 16 g/m², more preferably from 10 g/m² to 14 g/m². Exemplary backsheet films include those manufactured by Tredegar Corporation, based in Richmond, Va., and sold under the trade name CPC2 film. Other suitable backsheet materials may include breathable materials which permit vapors to escape from the absorbent article while still preventing exudates from passing through the backsheet (7). Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Some breathable composite materials are described in greater detail in PCT Application No. WO 95/16746 published on Jun. 22, 1995 in the name of E. I. DuPont; U.S. Pat. No. 5,938,648 to LaVon et al., U.S. Pat. No. 4,681,793 to Linman et al., U.S. Pat. No. 5,865,823 to Curro; and U.S. Pat. No. 5,571,096 to Dobrin et al, U.S. Pat. No. 6,946,585B2 to London Brown.

In a preferred embodiment the backsheet (7) is breathable. Breathable backsheet herein typically means that it comprises micro-openings sized to allow at least some water vapour to permeate through the backsheet that typically comprises a film such as a polyethylene (PE) film.

Preferably a backsheet (7) is breathable when the water vapour transmission rate (WVTR) of the backsheet is at least 500 grams/m2 - 24 hours, preferably at least 1,000 grams/m2 - 24 hours, even more preferably from 1,200 grams/m2 - 24 hours to 2,500 grams/m2 - 24 hours, even more preferably from 1,500 grams/m2 - 24 hours to 2,100 grams/m2 - 24 hours, as measured according to ASTM D6701-21.

The backsheet (7) may be joined to the topsheet (6), the absorbent core (8) or any other element of the absorbent article by any attachment means known in the art. Suitable attachment means are described above with respect to means for joining the topsheet to other elements of the article. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core (8) of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

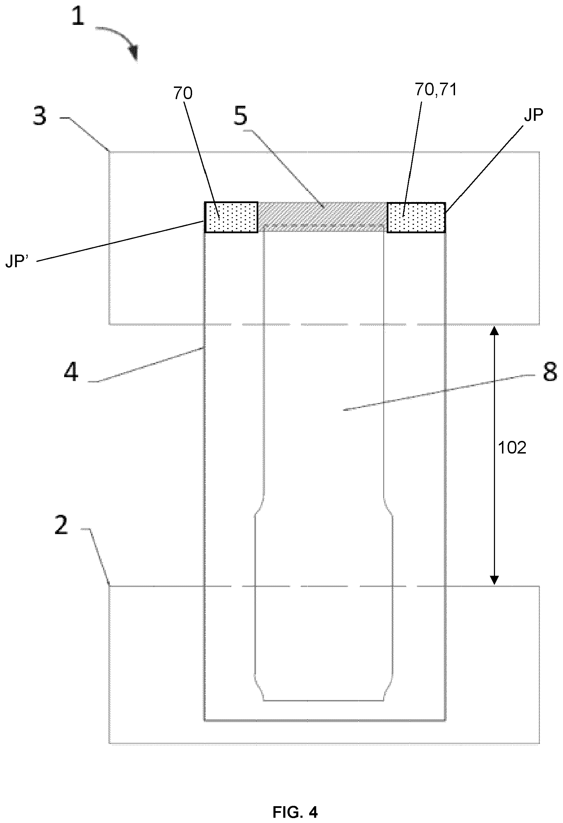

In an embodiment, as exemplified in Fig. 4, the transversal waist barrier(s) (5) overlap at least a portion of an absorbent core (8) of the absorbent insert (4) typically such that the exudates collected and/or retained by the transversal waist barrier(s) (5) are substantially dehydrated and/or absorbed by the neighbouring absorbent core (8) surface(s). Advantageously this allows for reduced leakage risks of highly liquid exudates like urine or liquid stool such as diarrhoea (or other liquid stool that is generally and commonly observed e.g. with new-born babies).

Preferably the front and back panels (2, 3) are separate and connected to each other by the absorbent insert (4) acting as a bridge element to form a substantially H-shaped pant. Advantageously this allows to omit cutting/shaping of panels in a crotch region of the article thus reducing waste and/or limiting the amount of panel material in view of its absence in the crotch region also helping to reduce waste and cost.

The absorbent insert (4) may be joined to the front and back panels (2, 3) by adhesive and/or mechanical bonding such as ultrasonic bonding, pressure bonding, and/or thermal bonding and the like. In a preferred embodiment the absorbent insert (4) is joined to the front and back panels (2, 3) by a discontinuous pattern of adhesive. Advantageously this may help to retain softness and pliability of the laminated structures and reduce stiffness when worn.

In an embodiment, the substantially liquid impermeable film layer comprises, preferably consists of, a backsheet (7) of the absorbent insert (4) and preferably comprises a material comprising, or consisting of, a polyester, more preferably said material being selected from the group consisting of polyethylene, polylactic acid, and polypropylene. Advantageously such transversal waist barrier(s) are not only impermeable to a much higher degree than hydrophobic nonwovens but further are formed by using a component already present in the absorbent article and hence does not require adding any further materials that would impact waste and/or cost.

Preferably, the at least portion of said absorbent insert (4) proximal to the front and/or back transversal edges thereof comprises one or more folds forming the transversal waist barrier(s) (5), preferably wherein said fold is substantially U-shaped. Advantageously such fold of the absorbent insert allows to form a liquid impermeable barrier to exudates, and particularly the U-shape (vs other shapes) allows to form a maximised pocket for exudate collection with the minimum use of material.

Preferably, the folded portion of said absorbent insert (4) is joined to the body-facing surface of the topsheet (6) at a pair of oppositely disposed extremities of the absorbent insert (4) along a portion of the left and right longitudinal edges thereof, generally wherein said oppositely disposed extremities are connected by a fold of said folded portion of said absorbent insert (4), such that a pocket is formed that acts as a barrier to exudates in both the longitudinal direction (i.e. substantially parallel to the longitudinal axis y) and the transverse direction (i.e. substantially parallel to the transverse axis x). The said extremities of the absorbent insert (4) may be joined by adhesive and/or mechanical bonding such as ultrasonic bonding, thermal bonding, and/or pressure bonding and the like.

In an embodiment, the absorbent insert (4) comprises a substantially liquid permeable topsheet (6), a substantially liquid impermeable backsheet (7), and an absorbent core (8) sandwiched therebetween, preferably wherein the absorbent core (8) is positioned inboard of the perimeter of said absorbent insert (4) when viewed in a planar direction (as generally depicted and illustrated in the figures herein and that typically corresponds to a plane formed by the x and y axis described and illustrated herein) such that the one or more folds are substantially free of absorbent material. Advantageously, a fold that is substantially free of absorbent material allows for a more flexible fold that can allow the barrier to better stand upright upon application of tension and yet limit piercing of the topsheet and/or backsheet that could arise if exerting a folding force in an area of the absorbent insert comprising SAP particles.

In an embodiment, the font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges, preferably such that said outer nonwoven layer (10) overlaps both a portion of the backsheet (7) and a portion of the topsheet (6) that remains exposed from said one or more transversal waist barrier(s) (5) such to form a flange (F_{L}). Advantageously this allows to cover the backsheet with nonwoven so that the backsheet does not come into contact with the skin of a wearer which could otherwise result in skin irritations and/or less comfort.

The inner and outer nonwoven layers (9, 10) may be the same or different meaning that they may have the same or different properties selected from basis weight (in gsm) and composition. In an embodiment, both the inner and outer nonwoven layers (9, 10) each comprise a spunbond nonwoven, typically comprising monocomponent fibers of polypropylene, and each have a basis weight of from 10 g/m² (gsm) to 30 g/m² (gsm), preferably from 12 g/m² (gsm) to 25 g/m². The outer nonwoven layer (10) may have a basis weight that is higher than the inner nonwoven layer (9). Advantageously, although it is customary to have the higher basis weight nonwoven on the inner nonwoven wearer skin facing side, in barrier embodiments herein (especially when the outer nonwoven layer is folded and/or extends over the absorbent insert; and/or when the transversal barrier is elasticised) such arrangement provides for added mechanical integrity desirable for resistance to tear on elastification and/or added softness for the wearer on the front and/or back waist regions up to the barrier location.

Preferably, and as exemplified in FIG. 1, the elastic material (11), such as the plurality of elastic strands, is/are deactivated in an area of overlap of the absorbent insert (4) when viewed in a planar direction. The deactivation is preferably achieved by cutting of the elastic strands such as to render the deactivated region of the panel(s) (2, 3) substantially inelastic and regions neighbouring said deactivated region and comprising said elastic strands being elastic. Typically the absorbent core (8) of the absorbent insert (4) is positioned within the deactivated region such that it substantially does not overlap the elastic strands (11). Advantageously this allows for improved core integrity as risks of core collapse due to excessive tension from the stretched elastics when the article is worn is limited.

Preferably more than 55%, preferably more than 60%, even more preferably from 65% to 95%, of the total surface area (generally when viewed in a planar direction) of the transversal waist barrier(s) (5) is positioned to overlap the deactivated region of the panel(s) (2, 3). Advantageously this allows for improved fit of the barrier when the article is worn by a subject.

In addition or alternatively, more than 50%, preferably more than 60%, even more preferably from 65% to 95%, of a total transversal length (generally substantially parallel to the transversal axis x) of the transversal waist barrier(s) (5) overlaps the deactivated region of the panel(s) (2, 3). Especially when the transversal waist barrier(s) is elastic, this arrangement may advantageously not only improve core integrity but further avoid excessive tension build-up.

In an embodiment, the transversal waist barrier(s) (5) is elastic, preferably comprising an elastic material selected from the group consisting of an elastic film and one or more elastic strands. Advantageously this allows for application of tension that permits the barrier to stand upright in close contact to the skin when the article is worn and under stretch.

The elastic strands in any of the embodiments herein may be strand-coated with adhesive prior to joining to one or more layers such as the inner and outer nonwoven layers (9, 10) to form elastic panels (2, 3) and/or the backsheet (7), outer cover (7'), and/or outer nonwoven layer (10) to form the elastic transversal waist barrier(s) (5). Advantageously this allows to join the one or more layers together by the said strand-coated elastic strands and by doing so limit the amount of adhesive used with resulting improved softness as well as reduced waste. Alternatively, or additionally, adhesive in the form of a plurality of stripes is applied such as by slot coating.

In a preferred embodiment, the one or more elastic strands of the transversal waist barrier(s) (5) comprise at least one flat elastic (such as elastics having a cross-section with an aspect ratio of longest to smallest dimension of greater than 1, preferably greater than 1.5, even more preferably greater than 2), preferably in combination with at least one or at least two round elastics (such as elastics having a cross-section with an aspect ratio of longest to smallest dimension of about 1). Although flat elastics are generally more expensive, they are advantageous in providing better gasketing effects and comfort on the wearer's skin, by combining flat and normal/round elastics one can achieve the desired gasketing and comfort whist limiting the cost.

When one or more, preferably only one, flat elastic is used, it is preferably positioned adjacent to an apex of the transversal waist barrier(s) (5) that may be closest to a transversal edge of the absorbent insert (4) and further round elastic(s) positioned further from said apex compared to the flat elastic(s). Advantageously this allows for the flat elastic to be positioned at the closest contact position to the wearer with other elastics being inboard (or further away) therefrom hence contributing to the desired tensile strength yet permitting to lower the overall density/dtex of the flat elastic and hence also cost, with limited impact on performance.

When elastic strands are used, they may have a dtex in the range of from 350 to 1100, preferably from 400 to 1000, even more preferably from 450 to 900. When elastic strands are used in all of the front and/or back panels (2, 3) and the transversal waist barrier(s) (5), the elastic strands of said panel(s) (2, 3) have a dtex that is equal to or greater than, preferably greater than, that of the elastic strand(s) of said transversal waist barrier(s) (5). Preferably the elastic strand(s) of said transversal waist barrier(s) (5) have a dtex that is from 40% to 85%, preferably from 45% to 75%, even more preferably from 50% to 70%, of the dtex of the elastic strands of said panel(s) (2, 3). Advantageously, this allows the barrier(s) to stand up and provide a gasketing effect close to the skin of the wearer without flattening or adding excessive resistance to stretch that would otherwise impact fit and comfort. The tension differential that results entails that a lower tension in the barrier(s) compared to the belt(s) is generated to a degree that upon stretching of the belt(s) the barrier(s) stand-up away from said belt(s).

The tensile stress (N/m) of the entirety of the front and back panels (2, 3), respectively, may be profiled in order to provide the functional benefits of the present disclosure, such as ease of stretch and application, while also maintaining certain force during wear, to prevent the article from sagging after loading. When the elasticity of the front and back panels (2, 3) are provided by a plurality of elastic strands (11) running in the transverse direction, the tensile stress may be adjusted by one or more of the following methods; 1) elongation rate of the elastic strands (11); 2) density (dtex) of the elastic strands (11); 3) longitudinal pitch of multiple elastic strands (11); and 4) effective length of elasticity of the elastic strands (11) in the transverse direction. By elongation, "0% elongation" is meant the original length of the elastic member.

The front and back panels (2, 3) may each be divided into multiple zones spanning in the transverse direction and defined by its location from the distal edge to the proximal edge relative to the percentage of the seam length wherein the distal edge is considered 0% and the proximal edge is considered 100%. The multiple zones may be configured to provide different tensile stress, or different functions to the front and back panels (2, 3), respectively.

In an embodiment, one or more second elastic strands (12) are comprised directly or indirectly between the outer nonwoven layer (10) and the backsheet (7), preferably between a body-facing surface of the outer nonwoven layer (10) and a garment-facing surface of the backsheet (7). An example of an indirect elastic arrangement is the presence of an outer cover layer (7') as described herein (that would generally be interposed between the backsheet (7) and the one or more second elastic strands (12), and an example of a direct elastic arrangement is the absence of an outer cover layer (7') as described herein. Advantageously this arrangement allows for a simple yet effective elastification of the barrier(s) permitting optimal gasketing effects.

Preferably, the one or more second elastic strands (12) extends along the entire transversal length of the transversal waist barrier(s) (5) and beyond, preferably crossing the imaginary longitudinal axis (y) in a direction substantially parallel to the imaginary transverse axis (x). Preferably the one or more second elastic strands (12) extends from a first position substantially adjacent a seam edge of the outer nonwoven layer (10) to a second position substantially adjacent an opposite seam edge of the outer nonwoven layer (10) with the imaginary longitudinal axis (y) being positioned therebetween, more preferably wherein the one or more second elastic strands (12) extends from said first position to said second position and crosses and/or overlaps the entire transversal waist barrier(s) (5) generally along the imaginary transverse axis (x). Advantageously this allows not only to form a standing barrier upon stretching but further aids better fit on the wearer's front and/or back waist.

In an embodiment, the absorbent core (8) comprises absorbent material selected from the group consisting of superabsorbent polymer particles and/or fibers; and cellulose fibers, preferably wherein said absorbent material is enclosed within a core wrap comprising top and bottom core wrap layers being a same nonwoven layer folded to sandwich the absorbent material therein or distinct nonwoven layers joined together to sandwich the absorbent material therein. The superabsorbent polymer particles and/or fibers may be comprised in an amount of greater than 50%wt, preferably greater than 60%wt, even more preferably greater than 70%wt, most preferably from 75% to 100%wt, by total weight of absorbent material.

In an embodiment, the back panel (3) is wider (generally in a direction substantially parallel to the longitudinal axis y) than the front panel (2) such that a length of the seams is substantially equal to a width (generally in a direction substantially parallel to the longitudinal axis y) of the front panel (2) and less than a width of the back panel (3); or wherein the front panel (2) is wider than the back panel (3) such that a length of the seams is substantially equal to a width of the back panel (3) and less than a width of the front panel (2). Advantageously this may permit comfort as well as improved protection compared to symmetric arrangements.

It is preferred that the backsheet (7) comprises a further outer cover layer (7') on a garment-facing surface thereof, preferably wherein said outer cover layer (7') comprising one or more nonwoven layers, and/or wherein the backsheet (7) comprises a further outer cover layer (7') that is shorter in the longitudinal direction (generally parallel to the longitudinal axis y) than the topsheet (6) and/or backsheet (7) such that at least a portion of an overlap length between the absorbent insert (4) and the panel (2, 3) is free of said outer cover layer (7') and preferably wherein the backsheet (7) is directly joined to, or is in contact with, the panel (2, 3) in said portion of an overlap length between the absorbent insert (4) and the panel (2, 3) free of said outer cover layer (7'). Advantageously this allows for improved softness in areas where needed and yet allow for material savings thus permitting both reduced cost and waste.

The outer cover layer (7') is preferably different from the inner and/or outer nonwoven layers (9, 10) meaning that they may have different properties selected from basis weight (in gsm) and composition. In an embodiment, the outer cover (7') comprises a spunbond nonwoven preferably comprising bicomponent fibers typically selected from the group consisting of polypropylene and polyethylene. The outer cover (7') preferably has a basis weight that is less than the basis weight of the inner and/or outer nonwoven layers (9, 10). The outer cover (7') may have a basis weight of from 8 g/m² (gsm) to 20 g/m² (gsm), preferably from

10 g/m² (gsm) to 15 g/m². Advantageously, the outer cover provides for softness to the touch to the care giver yet, as selected, it limits stress build-up particularly in embodiments with reduced length in the longitudinal direction as described above. Indeed without wishing to be bound by theory, stress build-up in the transition region corresponding to the terminal edge of the outer cover may result in a higher risk of tearing in the absorbent insert to panel joint.

In a preferred embodiment, the absorbent insert (4) is folded-over to form the waist barrier(s) (5) such that at least a portion of the topsheet (6) of the non-folded part of said absorbent insert (4) may come into contact with the folded part of said absorbent insert (4). Advantageously this allows the barrier to overlap the topsheet so as to help guide the exudates therethrough and into the core once stopped by said barrier.

Preferably, the waist barrier(s) (5) extends from 5% to 50%, preferably from 10% to 40%, even more preferably from 15% to 30%, of the total folded length (TFL) of the outer nonwoven layer (10) along the longitudinal axis (y). Advantageously, this may allow formation of a sufficiently large pocket yet avoid excessive resizing of the absorbent insert that may add cost and waste.

In an embodiment, the substantially liquid impermeable film layer or backsheet (7) comprises indicia, preferably a coloured print, at a position corresponding to the transversal waist barrier (5); and/or wherein the one or more second elastic strands (12) are coloured; and/or wherein a garment facing surface of the outer nonwoven layer (10) is coloured; and/or wherein the transversal waist barrier (5) comprises indicia selected from a coloured print and coloured adhesive (preferably comprising one or more pigments). Advantageously this allows the user or care giver to better visualise the barrier(s) so as to aid correct positioning and placement thereof onto a subject.

In an embodiment, the absorbent article further comprising a pair of longitudinally extending cuffs (105) positioned along left and right longitudinal edges of the absorbent insert (4), and wherein the transversal waist barrier(s) (5) is positioned above said cuffs such that said barrier(s) (5) is closer to a wearer's skin than said cuffs; preferably wherein the cuffs are joined to a body-facing surface of the topsheet (6) and wherein each of said cuffs is folded and joined to itself on a body-facing surface thereof at a joining position (JP) corresponding to the transversal waist barrier(s) (5), preferably said joints or joining positions (JP) extending along a lengthwise portion of, and being substantially adjacent to, the left and/or right longitudinal edges of the absorbent insert (4). Advantageously this reduces risk of leakage multidirectionally and prevents leakage to seep through between the transversal barrier and cuffs unlike when otherwise arranged.

Preferably the transversal waist barrier (5) is an up-standing barrier generally arranged to block substantially liquid exudates from flowing therethrough. Preferably, the liquid impermeable film layer is breathable and has a basis weight of less than 50 g/m², preferably from 8 g/m² to 45 g/m². The film may be elastic (or may comprise elastic material such as one or more elastic strands and/or elastic foam) and may comprise micro-openings sized to allow at least some water vapour to permeate therethrough. Preferably the film having a ratio of the MD load at break to the CD load at break of less than about 8, and a machine-direction notched trapezoidal tear strength of at least about 25 g.

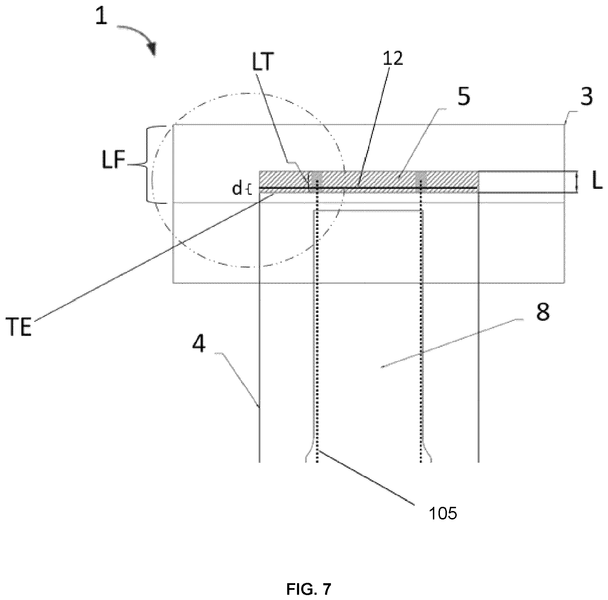

As shown in FIG. 7, in this embodiment, a portion of the absorbent insert proximal (typically adjacent) to the front and/or back transversal edges thereof may be folded onto itself and the fold (as described in embodiments herein) has a length (L) in a longitudinal direction running substantially parallel to a longitudinal axis (y), and wherein said absorbent insert (4) further comprises a pair of longitudinally extending cuffs 105 positioned along left and right longitudinal edges thereof and joined thereto along a tack-down length (LT) extending from a position proximal (preferably adjacent) to at least the back transversal edge to a tack-down terminal position longitudinally displaced therefrom along said longitudinal axis (y), and wherein the tack-down length (LT) is less than or equal to about 2L, preferably said cuffs 105 being joined to a body-facing surface of the topsheet (6). It is understood herein that whilst this embodiment makes specific reference to the "back" transversal edge, when the transversal waist barrier(s) (5) are positioned at the front of the article, the same dimensional characteristics apply with respect to the "front" transverse edge. Advantageously, this allows for a reduced tack-down length that permits to maximise the volume of the containment pocket formed by extending the lateral cavities when the fold stands up in contact with the user when the article is worn.

The back and/or front transversal edge (generally in folded state, as can be generally seen in the figures) typically coincides with a terminal edge (TE) of the transversal waist barrier (5) forming a pocket opening.

Preferably, the transversal waist barrier (5) comprises one or more second elastic strands (12) positioned at a distance (d) from a terminal edge (TE) of the transversal waist barrier (5) forming a pocket opening, and wherein the distance between the elastic closest to said terminal edge is less than about 0.6L, preferably from 0.15L to 0.5L, even more preferably from 0.20 to 0.45L. Advantageously this permits the transversal barrier to more easily stand-up and make a seal with the wearer's skin and preferably yet be sufficiently displaced from the terminal edge to minimize risk of elastic exposure to the skin of a wearer.

The font and/or back panels (2, 3) are elastic and comprise an inner nonwoven layer (9), an outer nonwoven layer (10), and an elastic material (11) sandwiched therebetween, wherein the elastic material is selected from the group consisting of an elastic film and a plurality of elastic strands. Preferably, the outer nonwoven layer (10) is wider than the inner nonwoven layer (9) (generally in a direction substantially parallel to the longitudinal axis y) and is folded over said inner nonwoven layer (9) such that it overlaps the portion of said absorbent insert (4) proximal to the front and/or back transversal edges and generally over a fold length (LF), preferably such that said outer nonwoven layer (10) overlaps both a portion of the backsheet (7) and a portion of the topsheet (6) that remains exposed from said one or more transversal waist barrier(s) (5) such to form a flange (F_{L}). Advantageously this allows to cover the backsheet with nonwoven so that the backsheet does not come into contact with the skin of a wearer which could otherwise result in skin irritations and/or less comfort.

The absorbent insert (4) may comprise a topsheet selected from an embossed nonwoven and/or a carded air-through-bonded nonwoven. The embossed nonwoven may comprise a spunbond nonwoven. Advantageously the topographical surface of such particular nonwoven selection allows for the formation of peaks and slumps that acts to not only slow down the movement of liquid stool towards the transversal edges but further allows for containment ridges in areas overlapping the barrier(s) (5).

Preferably, the thickness of the absorbent insert (4) in an overlap area of said waist barrier (5) (e.g. an area of the absorbent insert (4) overlapping with the waist barrier (5)) is less than the remaining thickness of said absorbent insert (4) (e.g. in areas other than the overlap area); and/or wherein the amount of absorbent material in in an overlap area of said waist barrier (5) is less (preferably at least 5%wt less, preferably at least 10%wt less, even more preferably at least 15%wt less, even more preferably at least 20%wt less) than the amount of absorbent material in other areas of said absorbent insert. Generally said overlap area further overlapping with the front and/or back panels respectively. Advantageously this allows for a larger pocket for exudate collection that synergistically cooperates with the overlapping front and/or back belts.

According to an embodiment the elastic material 11 sandwiched between the inner nonwoven layer 9 and the outer nonwoven layer 10 and the elastics strands 12 are different. The elastics 11,12 can differ in cross-sectional shapes, e.g. round or flat, in dtex, in composition and/or in color. Alternatively, said elastic material 11 and elastic strands 12 can be the same material.

The present disclosure pertains to a method to make such absorbent articles (1) as described hereabove. The method for the manufacture of an absorbent article (1) comprises the steps of:
- providing a front panel (2) comprising front and back transversal edges;
- providing a back panel (3) comprising front and back transversal edges;
- providing an absorbent insert (4) comprising front and back transversal edges and left and right longitudinal edges connecting the front and back transversal edges to form a perimeter thereof;
- folding a portion of the front panel (2) proximal to the front transversal edge and/or folding a portion of the back panel (3) proximal to the back transversal edge, about at least two folding lines to form a folded arrangement (64),
- joining said absorbent insert (4) to said folded arrangement and onto said elastic element,
- unfolding a portion of the folded arrangement (64) to form transversal waist barrier(s) (5); and
- joining said front and back panels (2, 3) together along a pair of oppositely disposed side seams.

Given that the absorbent insert (4) is joined to the folded arrangement, the unfolding of a portion of the folded arrangement (64) causes or leads to the folding of a portion of said absorbent insert (4) proximal to the front and/or back transversal edges thereof onto themself, preferably in the shape of a substantially U-fold. By "unfolding a portion of the folded arrangement (64)", it is meant opening one fold of the folded arrangement. In other words, the method further comprises the step of opening one fold (arrow C) of the folded arrangement (64), the folded arrangement (64) comprising at least two folding lines (62,63) and hence two folds.

This method is particularly well adapted for making such absorbent articles with transversal barriers, especially at high speeds when the front and/or back panels are provided as continuous web, the folding of the portion(s) of the absorbent insert (4) onto itself is carried out on a continuous web, i.e. the web of front and/or back panels, this stabilizes the folding process as it is done continuously. Advantageously an absorbent article having transversal barrier(s) as described herein can be attained in a simple yet effective manner. These steps are preferably carried out in this sequence, however some steps can be simultaneously or concurrent, for example the front and back panel can be provided simultaneously.

Another benefit of this method is that it enables to have a better control on the formation of said flange (F_{L}). Advantageously this allows to cover the backsheet with nonwoven so that the backsheet does not come into contact with the skin of a wearer which could otherwise result in skin irritations and/or less comfort.

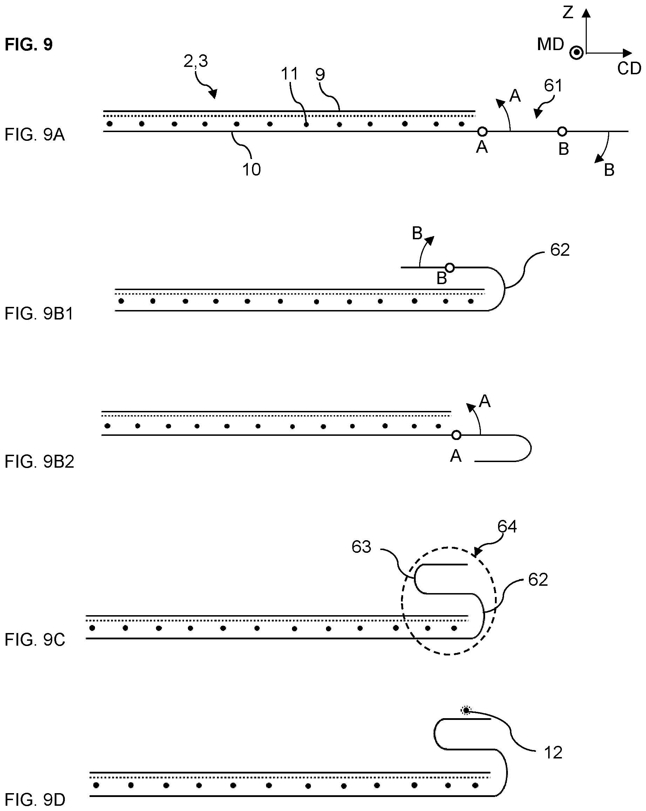

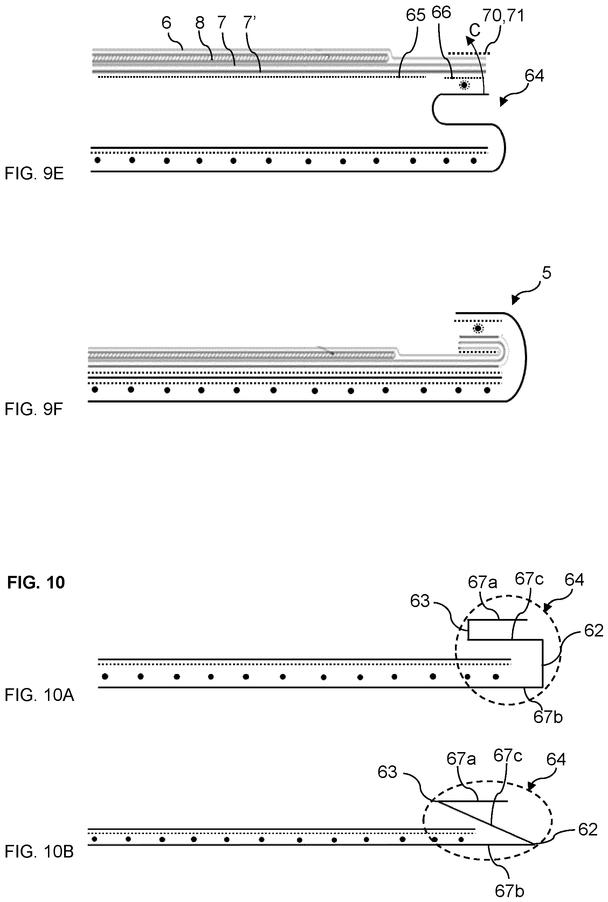

FIG. 9 (FIG. 9A to FIG. 9F) illustrates an embodiment of a folding arrangement, as described above, the folding arrangement (64) can be arranged on the front and/or back panel (2,3), meaning that the folding can be carried out on the web of front and/or back panel (2,3).

As illustrated in FIG. 9A, a web of front and/or back panel (2,3) is provided, said web comprising an inner nonwoven layer (9), an outer nonwoven layer (10) and elastic material (11), such as elastic strands, arranged in between. Said web preferably comprises an outboard portion (61) where the outer nonwoven layer (10) extends beyond the inner nonwoven (9) and elastic material (11) with respect to one direction, here the cross direction CD. A portion of the front and/or back panel (2,3), or a portion of the web(s) of front and/or back panel (2,3), proximal to the transversal edge is folded about a first folding line (62). Preferably, said outboard portion (61) is folded about a first folding line (62), or in other words, a portion of said outboard portion (61), namely a portion of the outer nonwoven layer (10) proximal to the front and/or back transversal edges is folded thereof onto itself, preferably in the shape of a substantially U-fold as shown in FIG. 9B. Hereunder, the method refers to the outboard portion (61) but the same folding concept can apply to a web of front and/or back panel (2,3) or to a front and/or back panel (2,3).

The first folding can be carried out about a first folding line (62) that can be proximal to the inner nonwoven layer (9) as shown in FIG. 9B1 where the folding is done at point A or distal to the inner nonwoven layer (9) as shown in FIG. 9B2 where the folding is done at point B.

The outboard portion (61) is then folded a second time about a second folding line (63) thereby forming a folded arrangement (64) as illustrated in FIG. 9C (dashed circle) where a portion of the web of front and/or back panel (2,3) proximal to the front and/or back transversal edges is folded twice thereof onto itself. More precisely, the outboard portion (61) of the outer nonwoven layer (10) of the web of front and/or back panel (2,3) is folded about at least two folding lines (62, 63) to form a folded arrangement (64).

The present disclosure is not limited to these embodiments, the two folding step can be done simultaneously or sequentially, the first folding step can be carried out as show in FIG. 9B1 or as shown in FIG. 9B2, meaning along a first folding line (63) (FIG. 9A : point B - arrow B) that is between the transversal edge of the outboard portion (61) or the front and/or back panel (2,3) and the portion of the outer nonwoven (10) that is attached to the inner nonwoven (9), with respect to the cross direction CD, or along a first folding line (62) (FIG. 9A: point A - arrow A) that is at the delimitation between the outboard portion (61) and the portion of the outer nonwoven (10) attached to the inner nonwoven (9). Of course, the first folding line (62) can also be arranged between the transversal edge of the outboard portion (61) or the front and/or back panel (2,3) and the portion of the outer nonwoven (10) that is attached to the inner nonwoven (9), with respect to the cross direction CD and still folded according to arrow A. Such folded arrangement are shown in FIG. 10 (FIG. 10A or FIG. 10B), where the first folding line (62) is remote from the portion of the portion of the outer nonwoven (10) attached to the inner nonwoven (9).

As illustrated in FIG. 9B1 and FIG.9B2, the notion of first and second folding line depends on where the first and second folding line is done and when in the process. For sake of clarity, the first folding line (62) is referenced as the folding line arranged distal to the transversal edge of the folded portion of the front and/or back panel (2,3), or outboard section (61), and the second folding line is reference as the folding line proximal to the transversal edge of the folded portion of the front and/or back panel (2,3), or outboard section (61).

According to an embodiment, as illustrated in FIG. 9D, the method comprises a further step of arranging an elastic material (12) onto or over the folded arrangement (4), namely a one or more second elastic material (12). As show in FIG. 9D, one second elastic material (12) is placed onto the folded arrangement (64).

As shown in FIG. 9E, the absorbent insert (4) comprising a substantially liquid permeable topsheet (6), a substantially liquid impermeable backsheet (7), and an absorbent core (8) sandwiched therebetween, the backsheet (7) comprising a further outer cover layer (7') on a garment-facing surface thereof is joined to the folded arrangement (64). The absorbent insert (4) can optionally be sprayed with a layer of adhesive comprising one or more sections or regions (65,66) such as a first region (65) ensuring the adhesion between the absorbent insert (4) and the inner nonwoven (9) of the front and/or back panel (2,3) and a second region (66) ensuring the adhesion between a portion of the absorbent insert (4) proximal to the transversal edge and the folded arrangement (64). Naturally, the adhesive can be sprayed or dispensed onto the web of front and/or back panels (2,3) instead or in combination. The first and second regions (65,66) can be connected, meaning forming continuity of matter, monolithic or the layer of adhesive being unitary, or said sections can be discrete, meaning separate or disconnected.

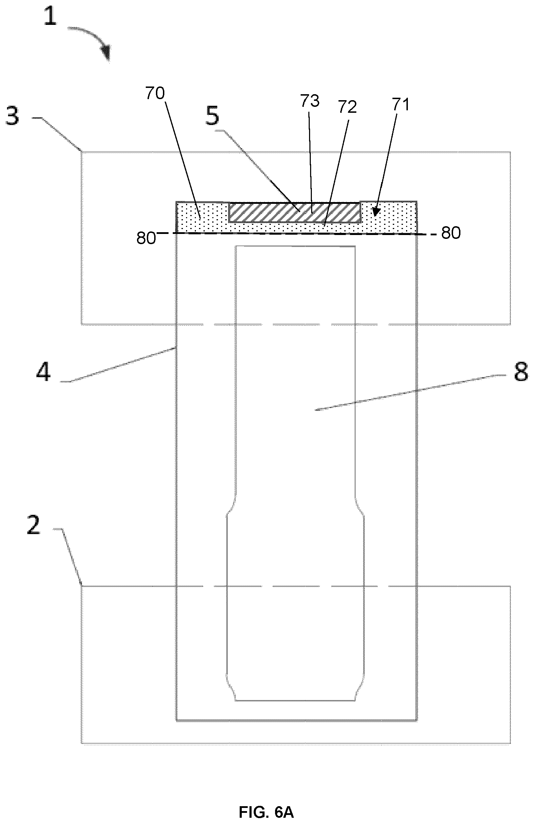

As shown in FIG. 9E, according to an embodiment, the method comprises the step of applying adhesive (70,71) onto the topsheet (6) and onto said portion of the absorbent insert (4) proximal to the front and/or back transversal edges in an adhesive pattern (71), said adhesive pattern (71) comprising at least two areas (70) of adhesive arranged at the transversal extremities of the front and/or back transversal edges as shown in FIG. 4. The adhesive pattern (71) can further comprise a further area (72) of adhesive extending transversally between said two areas (70) of adhesive, said further area (72) being at a longitudinal distance from the transversal edge, and an adhesive-free area (73) arranged between the transversal edge of the absorbent insert (4) and said further area (72) as shown in FIG. 6A.

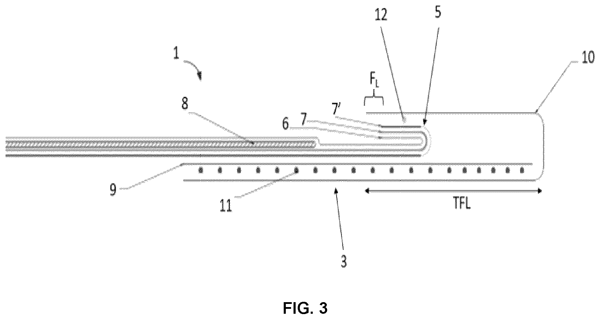

Still as shown in FIG. 9E, the method further comprises the step of unfolding (arrow C) a portion of the folded arrangement (64) to form transversal waist barrier(s) (5) as illustrated in FIG. 9F or in FIG. 3. In other words, the method further comprises the step of opening one fold (arrow C) of the folded arrangement (64), the folded arrangement (64) comprising at least two folding lines (62,63) and hence two folds.

FIG. 9 (FIG. 9A to FIG. 9F) comprises schematic drawings designed to illustrate the folding concept. There can be other representation such as in FIG. 10A or FIG. 10B both illustrating the folded arrangement (64) with two folding lines (62,63). As illustrated in FIG. 10A or 10B, the folded arrangement (64) comprises two folding lines (62,63) with a first panel (67a), corresponding to the portion of the front and/or back panel (2,3), or of the outboard section (61), arranged between the transversal edge and the second folding line (63), a second panel (67c) arranged between the two folding lines (62,63) and eventually a third panel (67b) arranged between the first folding line (62) and the portion of the outer nonwoven (10) that is attached to the inner nonwoven (9). As shown in FIG. 10A and 10B, the first panel (67a) is of lesser dimension than the second panel (67c), namely of lesser length with respect to the cross direction CD. The third panel (67b), when present, is preferably of lesser length than both first and second panels (67a,67c) with respect to the cross direction CD.

The folding arrangement (64) will now be further described hereunder. As illustrated above, the folded arrangement (64) comprises a Z-shaped fold. As indicated above, the folded arrangement (64) comprises two folding lines (62,63). The outboard portion (61) of the outer nonwoven (10) is folded twice in two opposite directions (arrow A and B).

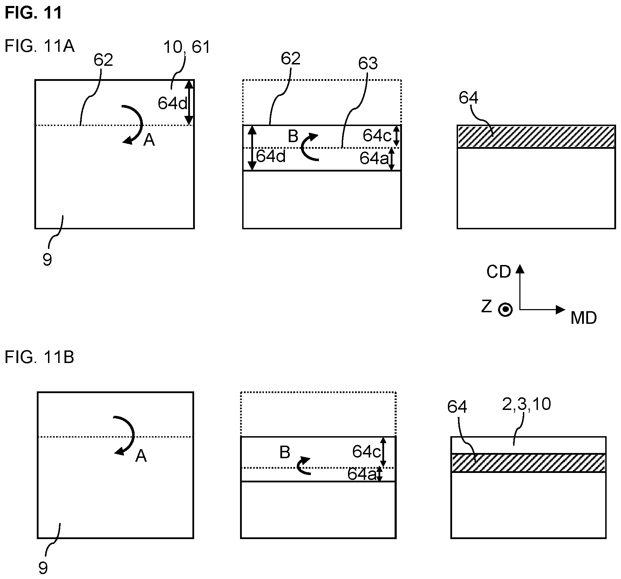

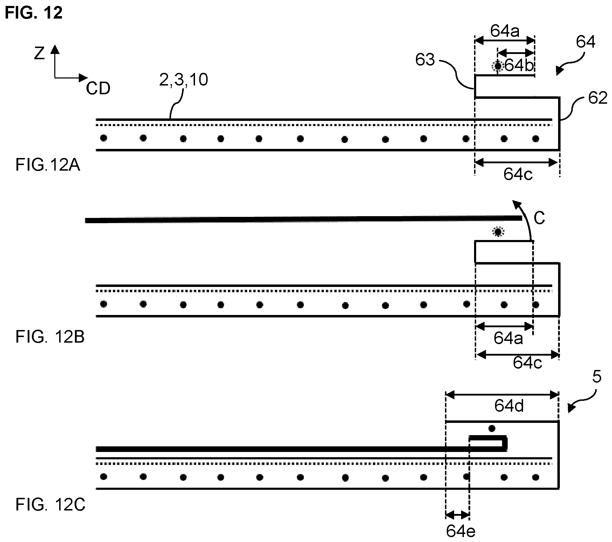

FIG. 11 illustrates another view of the folding concept, specifically a top view, meaning when looking at the web of front and/or back panel (2,3) from above. The outboard section (61) of the outer nonwoven (10) which extends beyond the inner nonwoven (9) with respect to one direction, such as the cross-direction CD, is folded (arrow A) about a first folding line 62. Meaning a portion of the outer nonwoven (10) proximal to the transversal edge is folded onto itself and eventually onto a portion of the inner nonwoven (9). This folded portion is then folded (arrow B) about a second folding line (63) to form a folded arrangement (64) in a Z-shaped fold. FIG. 9 and 11A illustrate folded arrangement where the folding is symmetric or even, meaning forming a Z-shaped fold where the second folding (arrow B) is in the center, with respect to the cross direction, of the folded portion of the outboard section (61), see middle drawing of FIG. 11A. As shown in FIG. 10 or 11B, the folded arrangement (64) can comprise asymmetrical or uneven Z-shaped folded where the second folding (arrow B) is not in the centre, with respect to the cross direction, of the folded portion of the outboard section (61). As explained hereunder and illustrated in FIG. 11A, the folded arrangement (64) can comprise a folding arrangement where the distances or length 64a and 64c are of equal value (symmetric). In other words, the first and second panel (67a,67c) can be of equal dimensions or length. As illustrated in FIG. 11B, the folded arrangement (64) can comprise a folding arrangement where distances 64a and 64c are of different values (asymmetric) with respect to the cross direction, preferably with distance 64c being greater than distance 64a, thereby leaving a strip of web of front and/or back panel outboard of the folded arrangement (64) with respect to the cross direction (CD) as illustrated in FIG. 12A, here the outer nonwoven 10 when viewing from above. ). In other words, the first and second panel (67a,67c) are or different dimensions or length, the second panel (67c) being of greater length than the first panel (67a). The present disclosure also pertains to embodiments where the first panel (67a) is of greater length than the second panel (67c).

FIG. 12 schematically illustrates the different dimensions in the folded arrangement (64) and of the transversal barrier (5) namely with respect to the cross-direction CD. In the top drawing of FIG. 12 (FIG. 12A), corresponding to the folded arrangement (64) before being joined to the absorbent insert (4), the length, or distance (64a) corresponds to the second folded portion or specifically the length, or distance between the second folding line (63) and the transversal edge of the outer nonwoven layer (10), the length, or distance (64b) corresponds to the distance between the one or more second elastic material (12) and the transversal edge of the outer nonwoven layer (10), the length, or distance (64c) corresponds to the unfolded portion of the outboard section (61) after the second folding or the distance between the first and second folding lines (62,63). In FIG. 12B, the middle drawing, the absorbent insert (4) is joined to the folded arrangement (4). In FIG. 12C, the bottom drawing, the folded arrangement is partially unfolded, meaning the folded portion of the outboard section is unfolded (arrow C) thereby leading the portion of the absorbent insert (4) proximal to the transversal edge to fold onto itself thereby forming a transversal barrier (5). The length, or distance (64d) corresponds to the folded portion of the outer nonwoven layer (9), or to the outboard section (61), or to the addition or combination of lengths, or distances (64a) and (64c). Finally, length, or distance (64e), corresponds to the length, or distance between the transversal edge of the absorbent insert (4) and transversal edge of the outer nonwoven layer (10) in the final folded configuration, meaning once folded onto themselves or once the transversal barrier (5) formed. In other words, the length, or distance (64e) corresponds to said flange (F_{L}) as illustrated in FIG.3. The terms "distance" or "length" are interchangeable terms meaning a dimension or spatial remoteness preferably in relation to one direction such as the cross direction CD.

According to an embodiment, length, or distance 64d is comprised between about 40 and 150 mm, preferably between about 50 and 100 mm, more preferably between about 70 and 85 mm. According to an embodiment, length, or distance 64c is comprised between about 20 and 100 mm, preferably between about 30 and 70 mm, more preferably between about 40 and 55 mm. According to an embodiment, length, or distance 64a is comprised between about 20 and 50 mm, preferably between about 25 and 40 mm, more preferably between about 30 mm. According to an embodiment, length, or distance 64b is comprised between about 10 and 30 mm, preferably between about 15 and 25 mm, more preferably between about 20 mm. According to an embodiment, length, or distance 64e is comprised between about 5 and 30 mm, preferably between about 6 and 20 mm, more preferably between about 10 mm. These dimensions each individually ensures a proper folding and a smooth formation of the transversal barrier (5).

According to an embodiment, the ratio between length, or distances (64d) and (64c) is comprised between about 0,4 and 7,5, preferably between about 0,7 and 3,3, more preferably between about 1,2 and 2,1. In other words, according to an embodiment, with respect to the cross direction (CD) the ratio (64d/64c) between the outboard section length or distance (61) and the length or distance between the first and second folding lines (62,63) is comprised between about 0,4 and 7,5, preferably between about 0,7 and 3,3, more preferably between about 1,2 and 2,1. These ratios ensure a proper formation of the transversal barrier (5).

As described above, preferably, the waist barrier(s) (5) extends from 5% to 50%, preferably from 10% to 40%, even more preferably from 15% to 30%, of the total folded length (TFL) of the outer nonwoven layer (10) along the longitudinal axis (y). Or in other words, the portion of the absorbent insert (4) proximal to the transversal edge folded thereof onto itself extends from 5 % to 50 %, preferably 10 % to 40%, more preferably 15% to 30%, of the distance or length (64d). Advantageously, this may allow formation of a sufficiently large pocket yet avoid excessive resizing of the absorbent insert that may add cost and waste.

The present disclosure is not limited to these embodiments. According to an embodiment, the method comprises a step of folding a portion of the front panel (2) proximal to the front transversal edge and/or folding a portion of the back panel (3) proximal to the back transversal edge, about four folding lines to form a folded arrangement, the folded arrangement being in the form of an omega-shaped folded formation.

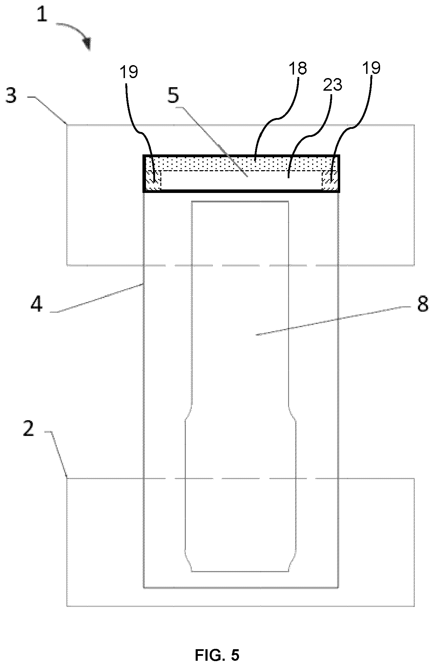

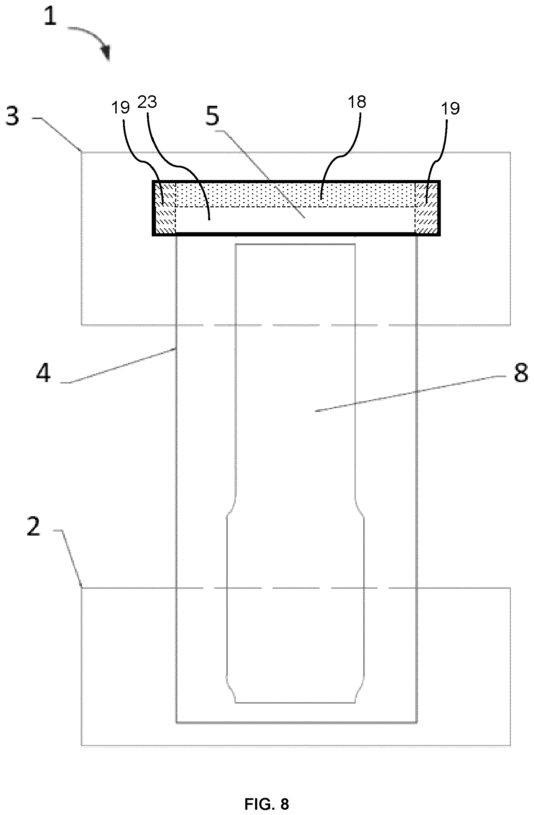

The transversal barrier can be formed with additional features described here above such as the method comprises the step of arranging an elastic material (12) over the folded arrangement (64) prior to step of joining said absorbent insert (4) to said folded arrangement (64) eventually further comprising the step of strand coating the elastic material (12) prior to the step of arranging said elastic material (12) over the folded portion of the folded arrangement (4). The method can further comprise the step of intermittently applying adhesive (70) onto said portion of the absorbent insert (4) proximal to the front and/or back transversal edges. The method can further comprise the step of intermittently applying adhesive onto said portion of the front panel (2) proximal to the front transversal edges and/or onto said portion of the back panel (3) proximal to the back transversal edges. The method can further comprise the step of applying adhesive onto said portion of the absorbent insert (4) proximal to the front and/or back transversal edges in an adhesive pattern (71), said adhesive pattern (71) comprising at least two areas (70) of adhesive arranged at the transversal extremities of the front and/or back transversal edges, and preferably a further area (72) of adhesive extending transversally between said two areas (70) of adhesive, said further area (72) being at a longitudinal distance from the transversal edge, and an adhesive-free area (73) arranged between the transversal edge of the absorbent insert (4) and said further area (72). The method can further comprise the step of compressing the folded portion of the folded absorbent insert (4) and folded arrangement, preferably at least the transversal waist barrier(s) (5), once formed. The method can further comprise the step of mechanically bonding the folded transversal edge of the absorbent insert (4) once the transversal waist barrier(s) (5) has been formed. For example, as illustrated in FIG. 5 or 8, adhesive is intermittently or continuously applied to a central portion 18 of the absorbent insert 4 proximal to the back transversal edge, then said portion is folded thereof onto itself, then the folded portion is mechanically bonded in portions 19 arranged at the lateral portion, meaning at each longitudinal ends, of said portion proximal to the transversal edge, e.g. by ultrasonic welding, to further secure the transversal waist barrier 5, the unbonded portion 23 serving as the transversal barrier 5.

Generally, the front and back panels herein are made from continuous webs that are subsequently severed into discrete front and back panels typically after the step of joining the absorbent insert 4 to the folded arrangement (64) and after the step of folding a portion of said absorbent insert 4 proximal to the front and/or back transversal edges thereof onto itself or unfolding a portion of the folded arrangement (64).

The present disclosure is not limited to this sequence of steps and the present disclosure encompasses different sequence of steps. For example, the one or more second elastic material (12) can be joined to the second region of adhesive (66) and then joined to the folded arrangement (64). The adhesive (70) applied intermittently can be applied on the topsheet (6) before joining the absorbent insert (4) to the folded arrangement (64).

Logically, to make an absorbent article 1 comprising a transversal barrier 5 arranged in the back, the method comprise a step of folding a portion of said absorbent insert 4 proximal to the back transversal edges thereof onto itself, to make an absorbent article 1 comprising a transversal barrier 5 arranged in the front, the method comprise a step of folding a portion of said absorbent insert 4 proximal to the front transversal edges thereof onto itself and to make an absorbent article 1 comprising two transversal barriers 5 arranged in the front and back, the method comprise a step of folding two portions of said absorbent insert 4 each respectively proximal to the front and back transversal edges thereof onto itself.

Preferably, said folding and unfolding steps are carried out by static folding where a static element, *i.e.* a stator or a stationary element such as a stationary rail or rod or plate or blade, deflects a portion of said webs of front and/or back panels (2,3) proximal to the front and/or back transversal edges thereof onto itself. In other words, the folding and unfolding steps are preferably done by passive folding meaning that the folding is done with a moving element, *i.e.* the webs of front and/or back panels (2,3), being folded by an unmoving, or immobile or fixed element, e.g. a rail or a rod or a blade. Such folding or unfolding is more cost efficient and more reproducible since it is continuous.

The present disclosure encompasses embodiments where the folding step is carried out by dynamic folding, where a mobile element, e.g. an actuated rail or rod or folding blade, moves or is actuated to fold a portion of said webs of front and/or back panels (2,3)proximal to the front and/or back transversal edges thereof onto itself. This can be beneficial if one wishes to change the dimensions or length (64a - 64e) during manufacturing of absorbent articles.

Preferably, the method further comprises the steps of folding an outer nonwoven layer 10 of the front and/or back panels 2, 3 over the folded absorbent insert 4 and/or an inner nonwoven layer 9 and joining said folded outer nonwoven layer 10 directly or indirectly to the folded absorbent insert 4. Advantageously this allows to prevent irritation or discomfort to the wearer as explained hereinabove whilst avoiding the use of added components/materials/elements whilst maintaining a simple and cost-effective production process.

Preferably, the method further comprises the step of arranging an elastic material 12 over or onto the folded portion of the folded arrangement (64) prior to step of joining the absorbent insert (4) to the folded arrangement (64). Advantageously this allows the transversal barrier(s) 5 to stand more upright.

Preferably, the method further comprises the step of intermittently applying adhesive onto said portion of the absorbent insert proximal to the front and/or back transversal edges. The adhesive can be applied in a uniform application, meaning in a uniform coating, or in swirls or in overlapping spirals. The adhesive can also be applied randomly meaning the fibers of the adhesive material are applied and arranged in a random configuration. For example, the adhesive can be applied using a spray nozzle in a spray nozzle application or the adhesive can be applied using a slot nozzle in a slot nozzle application or the adhesive can be applied using a roller in a roller application or the adhesive can be applied using a track applicator in a track application. Preferably, the adhesive is applied in a uniform spray application using a spray nozzle as such nozzles deliver consistent hot melt adhesive spray coatings, producing uniform patterns and repeatable bonds. The adhesive is applied in joining zones 70 or bonding zones that will be described hereunder. The joining or bonding zones 70 comprises at least one area where the adhesive or mechanical bonding means is/are applied.

According to an embodiment as illustrated in FIG. 4, which represents an absorbent article 1 after the folding step where a portion of said absorbent insert 4 proximal to the back transversal edges thereof is folded onto itself and where the adhesive was applied in the portions 70 located at the corners of the back transversal edge of the absorbent insert 4 but is substantially not applied on the portion where the absorbent core 8 extends.

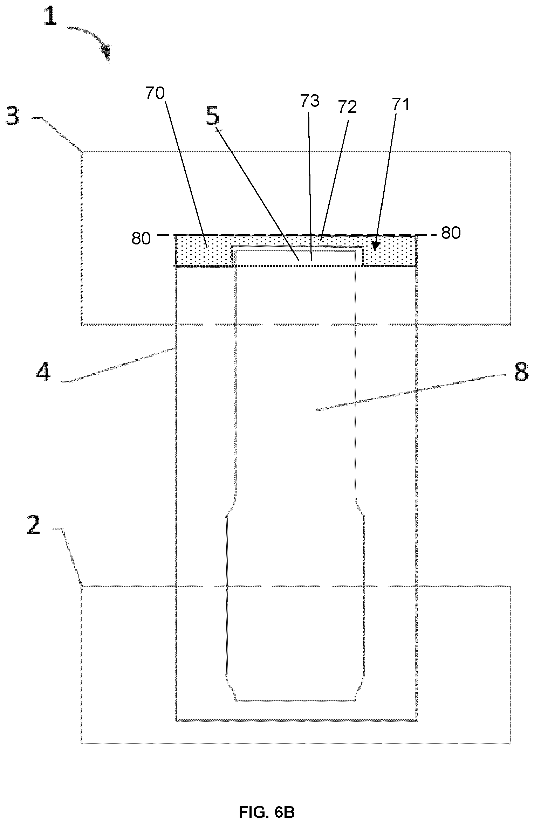

According to another embodiment as illustrated in FIG. 6A, which represents an absorbent article 1 prior to the folding of a portion of said absorbent insert 4 proximal to the back transversal edges thereof onto itself, the adhesive is applied in a pattern 71 on a portion of said absorbent insert 4 proximal to the back transversal edge. FIG. 6B illustrates the same absorbent article 1 after the folding step where the portion of the absorbent insert 4 proximal to the back transversal edge is folded thereof onto itself. The adhesive can also be applied in such pattern 71 to one portion of said absorbent insert 4 proximal to the front transversal edge. Naturally, the adhesive can be applied in such pattern 71 to both portions of said absorbent insert 4 proximal to the front and back transversal edges. The pattern 71 of adhesive comprises the two portions or two areas of adhesive 70, meaning two joining zones, as described hereabove, meaning the area 70 at the transversal extremities of the transversal edge, or in other words, the corners of the absorbent insert 4, or also the portions where the back transversal edge and longitudinal edges of the absorbent insert 4 connect as illustrated in FIG. 6A. The pattern 71 of adhesive further comprises a further area 72 of adhesive extending transversally connecting the two areas 70 and an area free of adhesive 73, said area free of adhesive 73 is arranged between the transversal edge of the absorbent insert 4 and the further area 72 of adhesive with respect to the longitudinal direction (y) and arranged between the two areas 70 at the corners with respect to the transversal direction (x). In other words, the pattern 71 of adhesive comprise a U-shaped pattern with two areas 70 corresponding to the upper branches of the U, said areas 70 being connected by a further area 72 corresponding to the lower branch of the U. Similarly, the pattern 71 of adhesive can comprise a H-shaped pattern with two areas 70 corresponding to the side branches of the H, said areas 70 being connected by a further area 72 corresponding to the middle branch of the H. The pattern of adhesive can comprise two areas 70 at the corners and a further area 72 of adhesive which extend only partially along the transversal axis (x) and does not connect the two areas 70 while still defining an area free of adhesive 73 as described above. In other words, the further area 72 can comprise a gap free of adhesive. In other words, the pattern 71 of adhesive can comprise two areas 70 that are L shaped in facing relationship or in mirror relationship, said L-shaped areas being arranged at each corner.

With an adhesive pattern 71 as described hereabove, when folding the absorbent insert 4 along the folding line 80 illustrated in FIG. 6A or FIG. 6B, a transversal waist barrier 5 is defined with the area free of adhesive 73 not being joined to the absorbent insert 4 and therefore standing upright. In other words, the barrier(s) 5 herein form an opening, typically about a terminal edge of the folded outer nonwoven layer, of an exudate containing pocket wherein substantially the entire surface of the pocket being congruent with a topsheet 6 of the absorbent insert 4 comprises a liquid impermeable layer preferably being a film, more preferably an elastic film (generally according to the embodiments described herein). With such adhesive pattern 71, the angle defined by the fold and the waist barrier is more acute and can provide a transversal waist barrier 5 with more resistance to exudates.

According to an embodiment, the method can comprise a mechanical bonding step where the portion of the absorbent insert 4 proximal to the back transversal edge is folded thereof onto itself and then mechanically bonded. The method comprises a step where the portion of the absorbent insert 4 proximal to the back transversal edge is glued to the absorbent insert 4 and/or a step where the portion of the absorbent insert 4 proximal to the back transversal edge is mechanically bonded to the absorbent insert 4. For example, adhesive is intermittently applied to the portion of the absorbent insert 4 proximal to the back transversal edge, then said portion is folded thereof onto itself, then the folded portion is mechanically bonded, e.g. ultrasonic welding, to further secure the transversal waist barrier 5.

The present disclosure pertains to an absorbent article (1) preferably manufactured by a method as described herein, wherein said article (1) comprises
- a substantially transversely extending front panel (2);
- a substantially transversely extending back panel (3); and
- a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof;

- wherein the front and back panels (2, 3) are joined together to define a pair of side seams, and
- at least a portion of said absorbent insert (4) proximal to the front and/or back transversal edges thereof comprises a transversal waist barrier (5) comprising a substantially liquid impermeable film layer,
- wherein the distance between the transversal edge of the absorbent insert (4) and transversal edge of the outer nonwoven layer (10) in the final folded configuration, meaning once folded onto themselves or once the transversal barrier (5) formed is comprised between about 5 and 30 mm, preferably between about 6 and 25 mm, more preferably between about 8 and 20 mm, even more preferably between about 9 and 15 mm.

Advantageously these dimensions in particular allow to cover the backsheet with nonwoven so that the backsheet does not come into contact with the skin of a wearer which could otherwise result in skin irritations and/or less comfort.

According to an embodiment, the shortest distance between the transversal barrier (5) and the second folding line (62) is comprised between about 20 and 100 mm, preferably between about 30 and 70 mm, more preferably between about 40 and 55 mm. This ensures that the transversal barrier (5) is remote enough from the edge of the absorbent article and hence avoid spill or blowout incidents.

The present disclosure also pertains to an apparatus to manufacture an absorbent article 1 as described herein and following the methods disclosed herein. The apparatus extends in a machine direction MD, a cross-direction CD and a vertical direction Z. The machine direction MD corresponds to the direction of travel of the webs, elastic materials or any components of the absorbent articles 1 being manufactured, the cross-direction CD is a direction perpendicular to the machine direction MD, and the vertical direction Z correspond to the height and is also a direction perpendicular to both the machine direction MD and the cross direction CD. In other words, the machine direction MD is the flow direction of an absorbent article and its components being assembled in the process line, e.g. the machine direction MD is the direction in which the absorbent insert 4 is conveyed. The terms "upstream" and "downstream" as used herein are with respect to the machine direction MD.

Said apparatus for the manufacture of an absorbent article (1) according to a method as described herein, comprises:
- a conveying device, preferably a conveyor belt or a plurality of rollers or drums, for conveying said front and/or back panels (2, 3);
- a folding unit for folding a portion of the front panel (2) proximal to the front transversal edge and/or folding a portion of the back panel (3) proximal to the back transversal edge, about at least two folding lines to form a folded arrangement,
- an unfolding unit for unfolding a portion of the folded arrangement and folding a portion of said absorbent insert (4) proximal to the front and/or back transversal edges thereof onto itself preferably in the shape of a substantially U-fold, to form transversal waist barrier(s) (5), said absorbent insert (4) being joined to said folded arrangement (64) and to front and/or back panel (2,3);
- a bonding unit for joining the joining said front and back panels (2, 3) together along a pair of oppositely disposed side seams

For sake of clarity, the coordinate system using longitudinal and transversal axis (x,y) pertains to the absorbent article 1 alone e.g. when laid flat on a surface, whereas coordinate system using machine, cross and vertical directions (MD, CD, Z) pertains to the apparatus for manufacturing absorbent article 1 with the absorbent articles 1 and their components being conveyed.

Following the methods described herein, a web of front panel 2 and a back panel 3 comprising the inner nonwoven layer 9 and outer nonwoven layer 10 as described hereabove, are conveyed, for example by a rotating roller, towards a first conveying device, e.g. a conveyor belt. The web of front and back panels 2,3 are assembled beforehand with the lamination of inner and outer nonwoven layers 9,10 and elastic material 11 and by intermittently severing the elastic material 11. The web of front and back panels 2,3 are for example conveyed with a gap arranged in between them, in other words, the front and back panels 2,3 are separated by a gap in the cross direction (CD) or in other terms, the web of front and back panels 2,3 are conveyed in the machine direction (MD) and/or vertical direction (V) and are at a distance of one another with respect to the cross direction (CD).. According to an embodiment, the front and back panels 2,3 are assembled together, meaning as one web, and are cut and separated prior to being joined to the absorbent insert 4 either individually or simultaneously.

The web of front and/or back panels (2,3) are conveyed toward a folding unit that will form the folded arrangement (64) by forming two folding lines (62,63). The folding unit may comprise two static, or stationary, elements that deflects said web(s) and induce a first fold and a second fold, the first static element being upstream of the second static element thereby forming a folded arrangement comprising a Z-shaped fold.

A strand coating unit can coat strand(s) of second elastic material (12) with adhesive and apply it onto the folded arrangement (64).

The absorbent insert 4 is deposited onto the folded arrangement and/or the inner nonwoven (9). The absorbent insert (4) is joined to the front and/or back panels, i.e. joined to the inner nonwoven (9) and folded arrangement (64), simultaneously or sequentially, thanks to the layer of adhesive comprising the first and second regions of adhesive (65,66) that is applied by a first adhesive dispensing device.

The front and back panels 2,3 and the absorbent insert 4 are conveyed towards a second adhesive dispensing device to apply adhesive on said absorbent insert 4. Said adhesive is applied, preferably intermittently, on at least one portion of said absorbent insert 4 proximal to the front and/or back transversal edges. The adhesive dispensing device comprises one nozzle and applies adhesive intermittently with respect to the machine direction onto the portion of said absorbent insert 4 proximal to the back transversal edge.

The adhesive is applied by the first or second adhesive dispensing device. Such application of adhesive may be by various processes such as slot coating, spray coating and other topical applications using a pneumatic, piezo or electromagnetic actuator. For example, the second adhesive dispensing device comprises at least one nozzle, each nozzle, for instance, may comprise a slot coating device or several exit ports and an adhesive flow control device such as electromagnetic valve, e.g. a solenoid valve. Preferably, the adhesive is applied in a uniform spray application using a spray nozzle as such nozzles deliver consistent hot melt adhesive spray coatings, producing uniform patterns and repeatable bonds. For example with an adhesive pattern 71 comprising two separate, or distinct or unconnected, areas of adhesive 70, e.g. arranged proximal to the corners of the back transversal edge of the absorbent insert 4 or the two L-shaped areas in facing relationship, a spray nozzle may be more adapted. With an adhesive pattern 71 comprising a continuous application of adhesive, such as a U-shaped or H shaped pattern, a slot nozzle may be more adapted.

The absorbent insert 4 and web of front and/or back panels (2,3) are then conveyed, or transported to an unfolding unit, where one fold of the folded arrangement (64) is opened, or the folded arrangement (64) is partially unfolded as illustrated in FIG. 9E . The unfolding unit preferably comprises a static element, meaning an element that is stationary and does not move such as a rail or a rod or a blade. In order to accelerate and improve the folding of a portion of the absorbent insert (4) thereof onto itself, the unfolding unit comprises a static element to further force said portion of the absorbent insert 4 proximal to the back transversal edge thereof to fold onto itself. In other words, the static element deviates the portion of the folded arrangement (64) and the portion of the absorbent insert 4 proximal to the back transversal edge thereof and facilitate and enhance the unfolding and folding motion so that the portion of the absorbent insert 4 proximal to the back transversal edge thereof can effectively fold onto itself.

Once the at least one portion of the absorbent insert 4 proximal to front and/or back transversal edge has been folded thereof onto itself thereby forming a transversal waist barrier 5, the absorbent insert 4 and front and back panels 2,3 can be conveyed toward one or more pressure roller(s) 146 which is arranged to press the folded portion of the absorbent insert 4, meaning the transversal waist barrier(s) 5, thereby securing the fold and eventually securing the glued and/or welded portions together.

The present disclosure is not limited to these embodiments, the method and process can comprise the placing of leg cuffs and leg elastics, wetness indicators, etc.

## Claims

1. Method for the manufacture of an absorbent article (1) comprising the steps of:
• providing a front panel (2) comprising front and back transversal edges;
• providing a back panel (3) comprising front and back transversal edges;
• providing an absorbent insert (4) comprising front and back transversal edges and left and right longitudinal edges connecting the front and back transversal edges to form a perimeter thereof;
• folding a portion of the front panel (2) proximal to the front transversal edge and/or folding a portion of the back panel (3) proximal to the back transversal edge, about at least two folding lines (62,63) to form a folded arrangement (64),
• joining said absorbent insert (4) to said folded arrangement (64),
• unfolding a portion of the folded arrangement (64) and folding a portion of said absorbent insert (4) proximal to the front and/or back transversal edges thereof onto itself, preferably in the shape of a substantially U-fold, to form transversal waist barrier(s) (5); and
• joining said front and back panels (2, 3) together along a pair of oppositely disposed side seams.

2. Method for the manufacture of an absorbent article (1) according to claim 1, wherein the folded arrangement (64) comprises a Z-shaped fold.

3. Method for the manufacture of an absorbent article (1) according to claim 2, wherein said Z-shaped fold comprises the transversal edge of the front and/or back panel (2,3) and a first and second folding lines (62,63), the length (64c) between the first and second folding lines (62,63) being greater than the length (64a) between the second folding line (63) and the transversal edge of the front and/or back panel (2,3), preferably with respect to the cross direction (CD).

4. Method for the manufacture of an absorbent article (1) according to claim 3, the Z-shaped fold comprises a total length (64d) corresponding to the addition of the length (64c) between the first and second folding lines (62,63) and the length (64a) between the second folding line (63) and the transversal edge of the front and/or back panel (2,3), preferably with respect to the cross direction (CD), wherein the ratio between the total length (64d) and the length (64c) between the first and second folding lines (62,63) is comprised between about 0,4 and 7,5, preferably between about 0,7 and 3,3, more preferably between about 1,2 and 2,1.

5. Method for the manufacture of an absorbent article (1) according to any of the preceding claims, further comprising the step of arranging an elastic material (12) onto the folded arrangement (64) prior to step of joining said absorbent insert (4) to said folded arrangement (64).

6. Method for the manufacture of an absorbent article (1) according to any of the preceding claims, further comprising the step of intermittently applying adhesive onto said portion of the absorbent insert (4) proximal to the front and/or back transversal edges.

7. Method for the manufacture of an absorbent article (1) according to any of the preceding claims, wherein prior to joining the absorbent insert (4) to the folded arrangement (64), the method further comprising the step of applying adhesive onto the garment facing side of the absorbent insert (4), said adhesive being applied in two discrete regions (65,66).

8. Method for the manufacture of an absorbent article (1) according to claim 6 or claims 6 and 7, further comprising the step of applying adhesive onto said portion of the absorbent insert (4) proximal to the front and/or back transversal edges in an adhesive pattern (71), said adhesive pattern (71) comprising at least two areas (70) of adhesive arranged at the transversal extremities of the front and/or back transversal edges, a further area (72) of adhesive extending transversally between said two areas (70) of adhesive, said further area (72) being at a longitudinal distance from the transversal edge, and an adhesive-free area (73) arranged between the transversal edge of the absorbent insert (4) and said further area (72).

9. Method for the manufacture of an absorbent article (1) according to any of the preceding claims, further comprising the step of compressing the folded portion of the folded absorbent insert (4) preferably at least the transversal waist barrier(s) (5), once formed.

10. Method for the manufacture of an absorbent article (1) according to claim 5 and claims 5 and 6 to 9, further comprising the step of strand coating the elastic material (12) prior to the step of arranging said elastic material (12) onto the folded arrangement (64).

11. Method for the manufacture of an absorbent article (1) according to any of the preceding claims, further comprising the step of mechanically bonding the folded transversal edge of the absorbent insert (4) once the transversal waist barrier(s) (5) has been formed.

12. Apparatus for the manufacture of an absorbent article (1) according to a method according to any of the claims 1 to 11, wherein the apparatus comprises:
- a conveying device, preferably a conveyor belt or a plurality of rollers or drums, for conveying said front and/or back panels (2, 3);
- a folding unit for folding a portion of the front panel (2) proximal to the front transversal edge and/or folding a portion of the back panel (3) proximal to the back transversal edge, about at least two folding lines to form a folded arrangement (64),
- an unfolding unit for unfolding a portion of the folded arrangement (64) and folding a portion of said absorbent insert (4) proximal to the front and/or back transversal edges thereof onto itself preferably in the shape of a substantially U-fold, to form transversal waist barrier(s) (5), said absorbent insert (4) being joined to said folded arrangement (64) and to front and/or back panel (2,3);
- a bonding unit for joining the joining said front and back panels (2, 3) together along a pair of oppositely disposed side seams.

13. Absorbent article (1) wherein said article comprises
- a substantially transversely extending front panel (2);
- a substantially transversely extending back panel (3); and
- a substantially longitudinally extending absorbent insert (4) joined to each of said front and back panels (2, 3), said absorbent insert (4) comprising front and back transversal edges and left and right longitudinal edges connecting the front and back transversal edges to form a perimeter thereof;
- wherein the front and back panels (2, 3) are joined together to define a pair of side seams, and
- at least a portion of said absorbent insert (4) proximal to the front and/or back transversal edges thereof comprises a transversal waist barrier (5) comprising a substantially liquid impermeable film layer,
**characterized in that** the length (64e,F_{L}) between the transversal edge of the absorbent insert (4) and transversal edge of the front and/or back panel (2,3) once the transversal barrier (5) has been formed is comprised between about 5 and 30 mm, preferably between about 6 and 25 mm, more preferably between about 8 and 20 mm, even more preferably between about 9 and 15 mm, preferably with respect to the cross direction.

14. Absorbent article (1) according to claim 13, wherein a portion of the front panel (2) proximal to the front transversal edge and/or a portion of the back panel (3) proximal to the back transversal edge comprises at least one folding lines (62), the shortest length (64c) between the transversal barrier (5) and said folding line (62) being comprised between about 20 and 100 mm, preferably between about 30 and 70 mm, more preferably between about 40 and 55 mm, preferably with respect to the cross direction.
